# EUROPÄISCHE PATENTANMELDUNG

(11) **EP 1 004 873 A1**
(43) Veröffentlichungstag der Anmeldung: **31.05.2000**
(21) Anmeldenummer: 99123161.4
(22) Anmeldetag: 22.11.1999
(51) Int. Cl.: G01N 21/64, G01N 33/58

(54) **Verfahren und Vorrichtung zur Identifikation der Funktion von biologischen Molekülen**

(30) Priorität: 24.11.1998 DE 19854195
(71) Anmelder: Xerion Pharmaceuticals GmbH, 82152 Martinsried (DE)
(72) Erfinder: Ng, Jocelyn, Dr., D-81369 München (DE); Jay, Daniel G., Dr., Brighton, MA 02135 (US); Ge, Liming, Dr., D-81455 München (DE); Ilag, Leodevico, Dr., D-81369 München (DE)
(74) Vertreter: Bösl, Raphael, Dr. rer. nat., Dipl.-Chem.

(57) **Zusammenfassung**

Die Erfindung betrifft ein Verfahren zur Identifikation der Funktion eines Liganden L, wobei die chromophor-unterstützte Laserinaktivierung (CALI) verwendet wird, gekennzeichnet durch die Stufen:
a) Auswählen eines Ligandenbindungspartners (LBP) mit Spezifität für den Liganden L,
b) Koppeln des LBP an einen laseraktivierbaren Marker (Tag) unter Bildung von LBP-Tag, gegebenenfalls nach vorheriger Modifikation des LBP im Hinblick auf eine effizientere Bindung an den Marker,
c) Inkontaktbringen des Liganden L mit mindestens einem LBP-Tag unter Bildung eines L/LBP-Tag-Komplexes, und
d) Bestrahlen des L/LBP-Tag-Komplexes mit einem Laserstrahl,
   wobei der bestrahlte LBP-Tag selektiv den gebundenen Liganden modifiziert,
   wobei die Reihenfolge der Stufen b) und c) vertauscht werden können.
Ferner betrifft die Erfindung eine Vorrichtung zur Durchführung des erfindungsgemäßen Verfahrens.

## Beschreibung

Die Erfindung betrifft ein Verfahren und eine Vorrichtung zur Identifikation der Funktion von biologischen Molekülen. Insbesondere betrifft die Erfindung ein Verfahren zur Identifikation der Funktion von biologischen Molekülen unter Verwendung der chromophor-unterstützten Laserinaktivierung (= chromophore-assisted laser inactivation; CALI). Das Verfahren beruht auf der spezifischen Bindung eines Liganden mit seinem Bindungspartner und dessen Inaktivierung durch das CALI-Verfahren.

Die vorliegende Erfindung betrifft das Gebiet der funktionellen Genomics und insbesondere betrifft sie ein Verfahren unter Verwendung von Ligandenbindungspartnern (LBP), bevorzugt ausgewählt aus kombinatorischen Banken oder gentechnisch erzeugt, um einen Zielliganden (L) zu modifizieren, um die Funktion des Zielliganden zu bestimmen.

Die Ausweitung des Gebiets der Genomics führte rasch zur Identifikation von vollständigen DNA-Sequenzen von Organismen. Es wird angenommen, daß das ganze menschliche Genom im Jahr 2003 vollständig sequenziert sein wird und die vollständige Strukturinformation entschlüsselt ist. Jedoch bietet die Kenntnis der DNA-Sequenz allein keine ausreichende Information, um zu verstehen, wie Gene und Krankheiten korrelieren, um die Entwicklung effizienterer Therapien gegen Krankheiten zu ermöglichen. Die Fülle von Informationen führte zur Entwicklung von neuen Technologien, um die Funktion dieser Gene besser zu verstehen. Gegenwärtig ist dies jedoch nicht möglich.

Das CALI-Verfahren betrifft die direkte Inaktivierung eines Proteins. Das CALI-Verfahren ist das aussichtsreichste Verfahren zur Bestimmung der Funktion eines Proteins. Mit Gen-Knockouts z.B. ist es nicht immer möglich, Mutationen für ein spezielles Protein zu erhalten und viele Tiersysteme sind genetischen Untersuchungen nicht leicht zugänglich. Bei den anderen Verfahren können hemmende Arzneimittel (Ribozyme und Antisense) nur für eine beschränkte Anzahl von Proteinen angewendet werden und funktionsblockierende Antikörper und Aptamere stellen nur einen kleinen Anteil derjenigen dar, die erzeugt werden. Andererseits kann CALI "Bindungsreagentien", wie Antikörper oder Liganden, in Funktionsblocker umwandeln. Kurz, eine Sonde (Ligandbindungspartner, LBP) wird mit dem Farbstoff Malachitgrün (MG) oder einem anderen Chromophor markiert, der nach Exposition gegenüber Laserlicht mit einer Wellenlänge, die von den zellulären Komponenten nicht signifikant absorbiert wird, freie Radikale erzeugt. Der mit MG markierte LBP (LBP-MG) wird mit der interessierenden Probe inkubiert. Ein Inaktivierungsbereich wird ausgewählt und mit einem Laserstrahl bei 620 nm bestrahlt. Das Licht wird von MG absorbiert, wodurch kurzlebige freie Radikal erzeugt werden, die an LBP-MG gebundene Proteine innerhalb eines Radius von 15 Å selektiv inaktivieren. Das System ist vielseitig, weil es für in vitro- und in vivo-Assays sowie für intrazelluläre und extrazelluläre Zielmoleküle verwendet werden kann.

CALI stellt ein vielversprechendes Werkzeug dar, um die Funktion der Gene zu erhellen, aber der Stand der Technik von CALI ist auf die Verwendung von ganzen Antikörpern (Jay, D.G. 1988, PNAS 85, 5454-5458) und Fab-Molekülen (Surrey, T. et al. 1998, PNAS 95, 4293-4298) beschränkt. Es wurde vorgeschlagen, daß Antikörpermoleküle aus dem Absuchen von monoclonalen Banken oder Hybridomen erzeugt werden können (Wang, F.-S. & Jay, D.G. 1996, Trends in Cell Biology 6, 442-445). Jedoch ist diese Variante extrem zeitaufwendig. Eine weitere Beschränkung ist, daß die ganzen Antikörper-Moleküle oder Fabs relativ groß sind und daher inaktiviert CALI möglicherweise das Protein in den Fällen nicht, in denen die Entfernung zwischen den MG-Bindungsstellen in dem Antikörper-Molekül und den für die Funktion des Proteins benötigten Domänen groß ist oder die Empfindlichkeit der Domäne gegenüber Schädigung durch Hydroxylradikale niedrig ist. Obwohl vorgeschlagen wurde, daß CALI mit jedem LBP angewendet werden kann, bestehen hierfür noch keine Anwendungsmöglichkeiten.

Verbesserungen für CALI wurden bereits vorgeschlagen (Surrey, T. et al. 1998, PNAS 95, 4293-4298). Es wurde gezeigt, daß anstelle von Malachitgrün das Fluoresceinmolekül als Chromophor verwendet werden kann. Fluorescein bietet den Vorteil, daß es löslicher und effizienter bezüglich der Inaktivierung ist, wobei eine geeignetere Lichtquelle mit kontinuierlicher Wellenlänge verwendet werden kann. Es wurde auch gezeigt, daß das Fab-Fragment in CALI-Experimenten funktionell ist. Jedoch wurde CALI so modifiziert, daß ein Epitop (d.h. Hämagglutinin (HA)) an jedes Zielmolekül von Interesse paßt und anschließende CALI-Experimente durchgeführt werden können. So dient nur ein Fab-Fragment (d.h. Anti-HA-Fab) als Ligand für alle Zielmoleküle, die durch CALI inaktiviert werden sollen.

Obwohl dieses Verfahren für Untersuchungen nützlich ist, bei denen keine nichtinaktivierenden Liganden verfügbar sind, die für kontrollierte Untersuchungen verwendet werden können, besitzt es mehrere Beschränkungen bei Anwendung auf dem Gebiet der funktionellen Genomics oder der Bewertung des Zielmoleküls. Zuerst ist das Verfahren sehr aufwendig, weil jedes Zielmolekül so manipuliert werden muß, daß es das spezifische Epitop einschließt. Ferner kann das eingeführt Epitop die Funktion des Zielmoleküls aufbrechen, wodurch die Einführung einer besser tolerierbaren Stelle in das Molekül erforderlich wird. Wenn jedoch diese besser tolerierbare Stelle zu weit von der funktionellen Stelle des zu untersuchenden Moleküls entfernt ist und sich außerhalb des Inaktivierungsbereichs befindet, funktioniert CALI jedoch nicht. Zweitens kann dies, obwohl Untersuchungen die Verwendbarkeit von Fab für CALI gezeigt haben, nicht verallgemeinert werden, weil Antikörper intrinsisch variieren. Es kann der Fall eintreten, daß der Chromophor eine günstige Befestigungsstelle in dem beschriebenen Anti-HA-Fab besaß, und dies für ein anderes Fab nicht zutrifft. Daher besteht eine Notwendigkeit, festzustellen, wo der Chromophor an das Anti-HA-Fab bindet und diesen zur Verallgemeinerung in andere Fabs hineinzumanipulieren. Alternativ können kleinere Liganden als Fabs benötigt werden, um CALI effizienter zu machen. Obwohl die Untersuchung weiter die Verwendung kleinerer Moleküle für CALI nahelegte, war sie auch auf das gleiche Molekülumfeld wie diese Anwendung beschränkt. Daher unterliegt sie den gleichen Beschränkungen, die überwunden werden müssen, damit CALI ein effektives Werkzeug für funktionelle Genomics und die Bewertung des Zielmoleküls ist.

Eine weitere Beschränkung von CALI als Verfahren zur Inaktivierung intrazellulärer Zielmoleküle ist der Prozeß der Abgabe des markierten Liganden in die Zelle. Obwohl existierende Methoden, wie Verreibung und Mikroinjektion, funktioniert haben, könnte dies auf bestimmte Zelltypen beschränkt sein. Jüngste Entwicklungen bei der Elektroporation können ebenfalls verwendet werden (Rols, M.-O. et al. 1998, Nature Biotechnology 16, 168-171).

Der vorliegenden Erfindung liegt daher die Aufgabe zugrunde, die gegenwärtigen Beschränkungen des CALI-Verfahrens zu modifizieren bzw. aufzuheben. Es sollen neue Verfahren bereitgestellt werden, die die Effizienz und Geschwindigkeit der Durchführung von CALI verbessern. Ebenso soll das Verfahren auf alle Bindungspartner anwendbar sein und automatisierbar sein. Das Verfahren soll eine einfache Bestimmung der Funktion von beliebigen Molekülen erlauben.

Die vorliegende Erfindung betrifft daher ein Verfahren zur Identifikation der Funktion eines Liganden L, wobei die chromophor-unterstützte Laserinaktivierung (CALI) verwendet wird, gekennzeichnet durch die Stufen:
a) Auswählen eines Ligandenbindungspartners (LBP) mit Spezifität für den Liganden L,
b) Koppeln des LBP an einen laseraktivierbaren Marker (Tag) unter Bildung von LBP-Tag, gegebenenfalls nach vorheriger Modifikation des LBP im Hinblick auf eine effizientere Bindung an den Marker,
c) Inkontaktbringen des Liganden L mit mindestens einem LBP-Tag unter Bildung eines L/LBP-Tag-Komplexes, und
d) Bestrahlen des L/LBP-Tag-Komplexes mit einem Laserstrahl,
   wobei der bestrahlte LBP-Tag selektiv den gebundenen Liganden modifiziert,
   wobei die Reihenfolge der Stufen b) und c) vertauscht werden können.

Überraschenderweise wurde gefunden, daß eine Kombination des CALI-Verfahrens mit geeigneten Verfahren zur Identifikation des Liganden eine rasche und einfache Bestimmung der Funktion eines Moleküls erlaubt. Bisher wurde angenommen, daß das CALI-Verfahren nur für bekannte Liganden mit vorbestimmten Spezifitäten wie z.B. Enzymsubstrate verwendet werden kann. Es war bisher nicht möglich, CALI in den Fällen anzuwenden, in denen keine bekannten Liganden verfügbar waren. Ferner war es bisher nicht möglich, CALI mit dem Screening von kombinatorischen Banken zu kombinieren.

Erfindungsgemäß wurde nun erstmalig CALI mit dem Screening von kombinatorischen Banken kombiniert. Erfindungsgemäß wurde gezeigt, daß es möglich ist, einen Liganden an einer spezifischen Stelle gezielt mit einem Chromophor zu markieren, wodurch die Möglichkeiten des CALI-Verfahrens deutlich vergrößert werden. Das Verfahren kann zur Identifikation der Funktion beliebiger Moleküle wie z.B. Proteine, Peptide, Kohlenhydrate, Lipide, DNA, RNA, etc. verwendet werden.

Erfindungsgemäß wird zunächst ein LBP mit Spezifität für den Liganden ausgewählt.

Mindestens ein ausgewählter LBP wird an einen Marker, bevorzugt einen laseraktivierten Marker gekoppelt, wodurch ein LBP-Tag gebildet wird. Die Kopplung von LBP mit dem Marker kann auch nach der Komplexbildung erfolgen. Dann wird L mit dem LBP-Tag in Kontakt gebracht, wodurch ein L/LBP-Tag-Komplex gebildet wird. Der L/LBP-Tag-Komplex wird mit einem Induktor in Kontakt gebracht, bevorzugt mit einem Laserstrahl bestrahlt, wodurch der Tag zu der selektiven Modifikation des gebundenen Liganden (L) führt.

Der LBP ist ein beliebiges Molekül, das geeignet ist, an einen Liganden zu binden. Bevorzugt ist er aus den folgenden Molekülen ausgewählt: scFv, Fab, Diabody, immunglobulinartige Moleküle, Peptid, RNA, DNA, PNA und kleine organische Moleküle, ausgenommen intakte Antikörpermoleküle. Gemäß einer weiteren bevorzugten Ausführungsform werden die vorstehend genannten LBPs aus einer kombinatorischen Bank nach einem der folgenden Verfahren, die einem Fachmann bekannt sind, ausgewählt: einstufige Selektion, (vgl. DE 19802576.9) Phagendisplay (Cwirla, S.E. et al. 1997, Science 273, 464-471), Peptide auf einem Plasmid (Stricker, N.L. et al. 1997, Nature Biotechnology 15, 336-342), SIP (Spada, S. et al. 1997, Biol. Chem. 378, 445-456), CLAP (Malmborg, A.-C. et al. 1997, JMB 273, 544-551), Ribosom/Polysom-Display (Kawasaki, G. 1991, internationale Patentanmeldung WO 91/05058; Hanes, J. & Pluckthun, A. 1997, PNAS 94, 4937-4942) oder SELEX (Tuerk, C. & Gold, L. 1990, Science 249, 505-510).

Bevorzugt stammt der Bindungspartner aus einer kombinatorischen Bank. Dies kann jede beliebige kombinatorische Bank sein, z.B. Proteinbank, Peptidbank, cDNA-Bank, mRNA-Bank, Bank mit organischen Molekülen, scFv-Bank mit Immunglobulinsuperfamilie, Proteindisplay-Bank etc.. In den Banken können präsentiert sein: alle Arten von Proteinen, z.B. Strukturproteine, Enzyme, Rezeptoren, Liganden, alle Arten von Peptiden einschließlich Modifikationen, DNAs, RNAs, Kombinationen von DNAs und RNAs, Hybride von Peptiden und RNA oder DNA, alle Arten von organischen Molekülen, z.B. Steroide, Alkaloide, Naturstoffe, synthetische Stoffe etc. Die Präsentation kann auf verschiedene Arten erfolgen, z.B. als Phagen-Display-System (z.B. filamentöse Phagen wie M13, fl, fd etc., lambda-Phagen-Display, virales Display etc.), Präsentation auf Bakterienoberflächen, Ribosomen etc.

Der aktivierbare Marker kann jedes beliebige Molekül sein, das kovalent oder nicht-kovalent an einen Bindungspartner gebunden werden kann und nach Induktion freie Radikale erzeugen kann. Beispiele hierfür sind photoinduzierbare Moleküle, z.B. Peroxidase mit Wasserstoffperoxid und laseraktivierbare Marker. Diese werden bevorzugt verwendet.

Gemäß einer bevorzugten Ausführungsform ist der laseraktivierbare Marker Malachitgrün, Fluorescein, Lissaminrhodamin, Tetramethylisothiocyanidrhodamin, Cyanin 3.18.
Weiterhin bevorzugt sind AMCA-S, AMCA, BODIPY und Varianten davon, Cascade Blue, Cl-NERF, Dansyl, Dialkylaminocumarin, 4',5'Dichlor-2',7'-dimethoxyfluorescein, DM-NERF, Eosin, Eosin F3S, Erythrosin, Hydroxycumarin, Isosulfan blue, Lissamine Rhodamin B, Malachitgrün, Methoxycumarin, Naphthofluorescein, NBD, Oregon Green 488, 500, 514, PyMPO, Pyrol, Rhodamin 6G, Rhodamin Green, Rhodamin Red, Rhodol green, 2',4',5',7'-Tetrabromsulfonfluorescein, Tetramethylrhodamin, Texas Red oder X-Rhodamin. Die Bestrahlung erfolgt mit Laserlicht einer Wellenlänge, die von dem jeweiligen Chromophor absorbiert wird.

Gemäß einer bevorzugten Ausführungsform werden der ausgewählte LBP oder jeder vorexistierende LBP so modifiziert, daß die Effizienz der Kopplung oder Bindung des Markers erhöht wird, wodurch ein effizienteres oder stabileres LBP-Tag-Molekül gebildet wird.

Beispielsweise können Lysinreste an das Protein addiert werden, wodurch Farbstoffmoleküle wie Malachitgrün oder Fluorescein über Isothiocyanatlinker leichter gekoppelt werden können. Es können auch bispezifische Moleküle nach an sich bekannten Verfahren erzeugt werden, wobei eine Spezifität z.B. gegen einen Chromophor, und die andere gegen den Liganden gerichtet ist.

Eine Variante ist die Erzeugung eines bispezifischen Moleküls mit Spezifitäten sowohl für den Liganden als auch den Marker. Zuerst wird ein scFv oder Ligand, der für den Tag spezifisch ist, über Phagendisplay, Peptide auf einem Plasmid, SIP, CLAP, Ribosom/Polysom-Display oder SELEX ausgewählt. Dieser spezifische Ligand für den Marker kann dann mit einer zweiten Domäne mit Spezifität für den Zielliganden (L) gekoppelt werden, wodurch ein Molekül mit zwei Spezifitäten erzeugt wird. Dies kann mit Verfahren, die einem Fachmann auf dem Gebiet bekannt sind, wie beispielsweise mittels Diabodies (Holliger, P. et al. 1993, PNAS 90, 6444-6448) durch die Verwendung einer Domänenassoziation, wie Fos-Jun-Leucinzipper (O'Shea, E.K. et al. 1989, Science 245, 645-648; Kostenly, S.A. et al. 1992, J. Immunol. 148, 1547-1553), einkettige chimäre Moleküle mit zwei Spezifitäten (Gruber M. et al. 1994, J. Immunlology 152, 5368-5374); Mack, M. et al. 1995, PNAS 92, 7021-7025) oder chimärem SELEX durchgeführt werden. Ferner kann LBP gentechnisch erzeugt werden, so daß es Aminosäuren (z.B. Cys und Lys) enthält, die leicht einer chemischen Verknüpfung mit dem Chromophor zugänglich sind (Hudson, L. & Hay, F.C. 1976, Fluorescein Conjugation technique. In Practical Immunology. Blackwell Scientific Publications, Boston, Kapitel 2.1.1, 14-17; Jay, D.G. 1988, PNAS 85, 5454-5458; Surrey, T. et al. 1998, PNAS 95, 4293-4298). Dabei werden im Handel erhältliche Verbindungen verwendet.

Gemäß einer bevorzugten Ausführungsform kann, wenn ein LBP ein ganzes Antikörpermolekül ist, dies in ein scFv oder Fab durch rekombinante Verfahren, die jedem Fachmann auf dem Gebiet bekannt sind, umgewandelt werden (Clackson, T. et al. 1991, Nature 352, 624-628; Huston, J.S. et al. 1988, PNAS 85, 5879-5883).

Gemäß einer weiteren Ausführungsform wird der LBP gentechnisch so manipuliert, daß er Aminosäuresequenzen enthält, die einen effizienten Transport von LBP in die Zellen erlauben, um eine breitere Anwendbarkeit von CALI auf ein breites Spektrum von Zelltypen zu ermöglichen und die Effizienz für intrazelluläre Zielmoleküle zu erhöhen. Es wurde gezeigt, daß kurze Peptidsequenzen (Rojas, M. et al. 1998, Nature Biotechnology 16, 370-375) und das VP22-Protein des Herpesvirus (Phelan, A. et al. 1998, Nature Biotechnology 16, 440-443) in Fusion an heterologe Proteine zu einem effizienten Transport in Säugerzellen führen. So können beispielsweise Signalpeptidsequenzen oder Homeodomänen, die den Transport großer Proteinmoleküle in die Zellen erleichtern, mit den gewünschten Bindungspartnern durch gentechnische Fusion oder durch ein chemisches Kopplungsverfahren verknüpft werden. Derartige Verfahrensschritte sind einem Fachmann auf dem Gebiet gut bekannt. Für die chemische Kopplung sind einen Fachmann ebenfalls entsprechende Verfahren bekannt, z.B. Kopplung über SH-geschützte Cysteine.

Der Ligand und der LBP-Tag werden unter Bedingungen in Kontakt gebracht, unter denen eine Komplexbildung erfolgt. Derartige Bedingungen wurden bereits bei der Erforschung der Ligandenwechselwirkungen, Mobilitätstests etc. erforscht und sind einem Fachmann gut bekannt. Bevorzugt sind dies physiologische Bedingungen. Die Komplexbildung zwischen dem Liganden und den markierten Bindungspartner erfolgt sehr spezifisch und wird durch begleitende Prozesse oder Komplexe nicht gestört.

Die Identifikation des modifizierten Liganden erfolgt mit einen geeigneten chemische/physikalischen Verfahren.

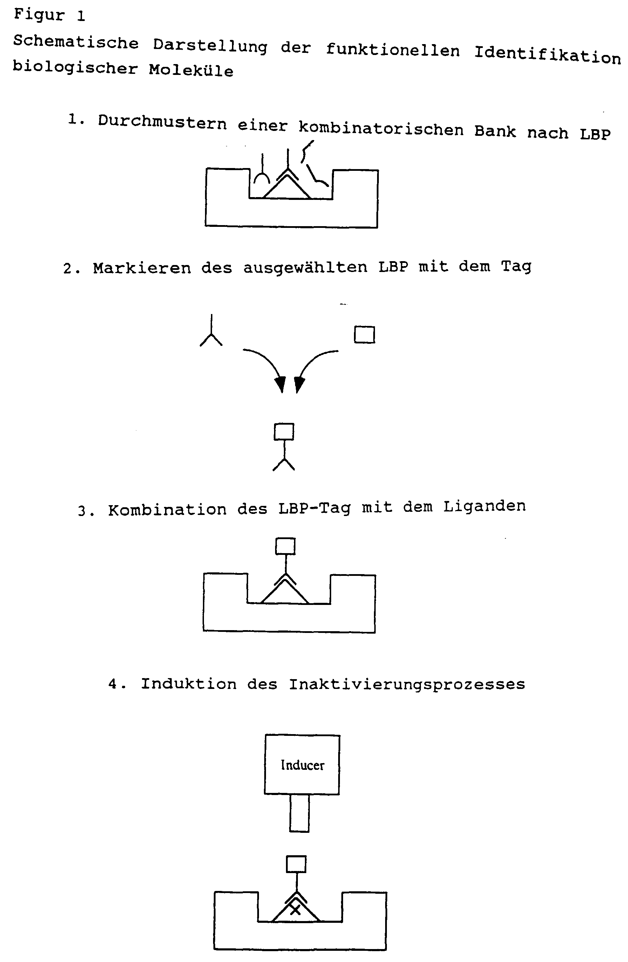

Die beigefügte Figur 1 zeigt das erfindungsgemäße Verfahren im Überblick.

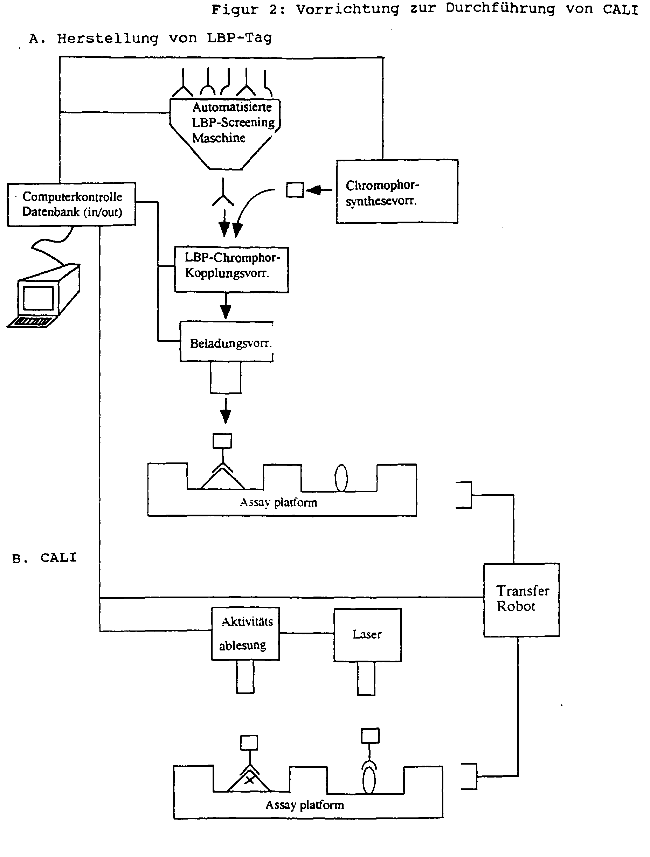

Die Erfindung betrifft auch eine Vorrichtung zur Durchführung des erfindungsgemäßen Verfahrens. Diese Vorrichtung ist in der beigefügten Figur 2 näher dargestellt. Diese Vorrichtung ist ein automatisiertes System aus integrierten unabhängigen Einheiten/Teilen zur Identifikation der Proteinfunktion. Teil A betrifft die Herstellung von LBP-Tag.
Eine automatisierte LBP-Screeningmaschine ergibt spezifische LBPS, die gegen spezielle Zielmoleküle/Liganden gerichtet sind, während die Chromophor-Synthesevorrichtung Chromophore der Wahl produziert. Die ausgewählten LBPs und die synthetisierten Chromophore werden chemisch in einer LBP-Chromophor-Kopplungsvorrichtung verknüpft, wodurch der LBP-Tag erhalten wird. Dieser LBP-Tag wird in eine Beladungsvorrichtung überführt, die den LBP-Tag in vorbestimmte Kavitäten überführt, die mit dem Zielmolekül/Liganden in der Assay-Platform beschichtet sind. Ein Transferroboter bewegt dann die Assay-Platform in das Laser-System, um den zweiten Teil B zu initiieren. Die Proben werden mit dem Laser in der gewünschten Wellenlänge bestrahlt, um eine Modifikation durch freie Radikale zu induzieren. Eine Vorrichtung zur Ablesung der Aktivität fährt dann über die Kavität, die mit Laser bestrahlt worden ist, um die biologische oder chemische Aktivität der bestrahlten Proben aufzuzeichnen.
Alle Teile der Vorrichtung sind mit einem Zentralcomputersystem zur Kontrolle und Analyse verbunden.

Die Erfindung wird anhand der folgenden Beispiele näher erläutert.

### Beispiel 1

### Selektion von scFv-Antikörpern und CALI von β-Galactosidase

### i) Selektion von Anti-β-Galactosidase-scFv-Antikörpern mittels Phagendisplay.

Eine Phagendisplaybank aus menschlichen einkettigen Antikörpern (scFv) wurde, wie vorstehend beschrieben (Sheets, M.D. et al. 1998, PNAS, 95:6157-6162; Marks, J.D. et al. 1991, J. Mol. Biol. 222:581-597) hergestellt. ScFv-Antikörper, die für β-Galactosidase spezifisch sind, wurden aus dieser Bank durch Phagendisplay und Biopanning mit Zuvor beschriebenen Methoden isoliert (Sheets, M.D. et al. 1998, PNAS, 95:6157-6162; Marks, J.D. et al. 1991, J. Mol. Biol. 222:581-597). 50 µg gereinigte β-Galactosidase wurden in Vertiefungen einer ELISA-Platte beschichtet und zur Selektion mittels Phagendisplay verwendet. Drei Runden Panning wurden durchgeführt und die angereicherten Phagen, die einen scFv mit Spezifität für β-Galactosidase enthielten, wurden in einen Extraktionsvektor (Marks, J.D. et al. 1991, J. Mol. Biol. 222:581-597) subcloniert und auf die Spezifität für β-Galactosidase mittels ELISA und durch ihre Fähigkeit, die β-Galactosidaseaktivität zu hemmen, getestet (Wallenfells, K., 1962, Methods Enzymol. 5:212-219). Als negative Kontrolle wurde ein Anti-Testosteron-scFv verwendet.

Mehrere scFvs, die die β-Galactosidase-Aktivität nicht hemmten oder eine ähnlich β-Galactosidaseaktivität hatten wie die negative Kontrolle wurden für den nächsten Schritt verwendet.

### ii) Markierung der scFv-Antikörper mit Fluorescein und Malachitgrün

Die vorstehend erhaltenen scFvs (die die β-Galactosidaseaktivität nicht hemmen) gegen β-Galactosidase wurden mit Malachitgrünisothiocyanat oder Fluoresceinisothiocyanat mit Modifikationen, wie beschrieben (Jay, D.G. 1988, PNAS 85: 5454-5458; Surrey, T. 1998, PNAS 95:4293-4298), markiert. Kurz ausgedrückt, Antikörper in einer Konzentration von 600 µg/ml in 500 mM NaHCO₃ (pH 9,8) wurden stufenweise durch Zugabe von Malachitgrünisothiocyanat oder Fluoresceinisothiocyanat (aus Molekularsonden, Eugene, OR) bis zu einer Konzentration von 120 µg/ml aus einer Stammlösung (20 mg/ml oder 2 mg/ml) in DMSO markiert. Nach einstündiger Inkubation bei Raumtemperatur unter Rühren oder vierstündiger Inkubation auf Eis wurde die Lösung durch eine Entsalzungsäule in 150 mM NaCl/50 mM NaPi, pH 7,3 (für Malachitgrün muß das Präzipitat zentrifugiert werden, bevor der Puffer gewechselt wird) geleitet, um den Marker von dem markierten Protein abzutrennen.
Die markierten Antikörper wurden isoliert und auf die Anwesenheit des Chromophors mittels UV-Absorption getestet. CALI von β-Galactosidase

### iii) CALI von ß-Galactosidase

Für CALI erfolgt der Einsatz des Lasers sowie die Beleuchtung mit einem Laserstrahl im wesentlichen, wie beschrieben (Beerman, A.E. und Jay, D.G. 1994, Methods Cell Biol. 44, 715-732; Surrey, T. 1998, PNAS 95:4293-4298). Eine 20 µl Probe, die β-Galactosidase (10 µg/ml), farbstoffmarkierte Antikörper gegen β-Galactosidas (200 µg/ml) aus ii) enthielt, wurde auf eine ELISA-Platte gegeben. Das Gesamtvolumen der Vertiefung wurde mit einem Laserstrahl nach verschiedenen Zeitspannen beleuchtet. Die Aktivität der Proben wurde, wie vorstehend beschrieben, gemessen (Wallenfells, K. 1962, Methods Enzymol. 5:212-219). Die negativen Kontrollen bestanden aus einem scFv-Chromophor/β-Gal-Komplex, der nicht mit Laser bestrahlt worden war, sowie einem CALI-Experiment mit Anti-Testosteron scFv.

Die CALI-Untersuchtung mit β-Gal mit β-Gal-spezifischeen scFvs führte zu einer Inaktivierung von mehr als 90%, verglichen mit den negativen Kontrollen

Die Aktivität der Beta-Galactosidase nach CALI wurde als Funktion der zeit verfolgt. Die Aktivität wurde als Einheiten gemessen, die dem Test entsprechen und als Verhältnis zu der Aktivität der unbehandelten Beta-Galactosidase ausgedrückt sind.

| CALI von Beta-Galactosidase | | |
|---|---|---|
| Zeit (min) | % aktive Beta-Galactosidase | |
| | mit CALI | ohne CALI |
| 0 | 100 | 100 |
| 1 | 100 | 100 |
| 3 | 75 | 100 |
| 5 | 22 | 100 |
| 10 | 5 | 100 |
| 30 | <1 | 100 |

### Beispiel 2

### Selektion von Antameren und CALI von β-Galactosidase

### i) Selektion der Aptameren, die für β-Galactosidase spezifisch sind, mittels SELEX

DNA-Aptamere, die für β-Galactosidase spezifisch sind, wurden aus einer Zufalls-DNA-Bank mittels SELEX mit zuvor beschriebenen Methoden (Morris, K.N. et al. 1998, PNAS 95:2902-2907) isoliert. Verschiedene Konzentrationen (0,1 bis 2 mg/ml) β-Galactosidase wurden zur Selektion verwendet. Nach 25 Selektionsrunden wurden die angereicherten DNA-Aptamere sequenziert und einzeln auf Spezifität gegen β-Galactosidase und die Fähigkeit, die β-Galactosidasaktivität zu hemmen, getestet (Wallenfells, K. 1962, Methods Enzymol. 5:212-219). Als negative Kontrolle wurden Aptamere, die für das U1A-Protein spezifisch sind, in den β-Galactosidase-Hemmtests verwendet.

Mehrere Aptamere, die die β-Galactosidase nicht hemmten oder eine ähnliche β-Galactosidaseaktivität hatten, wie die negative Kontrolle, wurden für den nächsten Schritt ausgewählt.

### ii) Markierung von Nucleotidaptameren mit Fluorescein und Malachitgrün

Die ausgewählten DNA-Aptameren, die die β-Galactosidaseaktivität nicht hemmten, wurden zur Markierung mit Fluorescein und Malachitgrün verwendet. Mehrere Verfahren einschließlich sowohl enzymatischer als auch chemischer Synthese, die einem Fachmann auf dem Gebiet bekannt sind, wurden verwendet, um farbstoffmarkierte Nucleotide als Teil des Aptameren einzuschleusen (Igloi, G.L. 1996, Anal. Biochem. 233:124-129; Meyer, K.l: & Hanna, M.M. 1996, Bioconjug. Chem. 4:401-412; Nelson, P.S. et al. 1992, Nucleic Acids Res. 20:6253-6259; Theisen, P. et al. 1992, Nucleic Acids Symp. Ser 27:99-100; Proudnikov, D. & Mizabekov, A. 1996, Nucleic Acids Res. 24:4535-4542; Rosemeyer, V. et al. 1995, Anal. Biochem. 224:446-449; Richardson, R.W. & Gumport, R.I. 1983, Nucleic Acids Res. 11:6167-6184; Kinoshita, Y. et al. 1997, Nucleic Acids Res. 25:3747-3748).

Die markierten Aptameren wurden mit Polyacrylamidgelelektrophorese isoliert und auf die Anwesenheit des Chromophors mittels UV-Absorption getestet.

### iii) CALI von β-Galactosidase

Für CALI erfolgt der Einsatz des Lasers sowie die Beleuchtung mit einem Laserstrahl im wesentlichen, wie beschrieben (Beerman, A.E. und Jay, D.G. 1994, Methods Cell Biol. 44, 715-732; Surrey, T. 1998, PNAS 95:4293-4298). Eine 20 µl Probe, die β-Galactosidase (10 µg/ml), farbstoffmarkierte Antikörper gegen β-Galactosidas (200 µg/ml) aus ii) enthielt, wurde auf eine ELISA-Platte gegeben. Das Gesamtvolumen der Vertiefung wurde mit einem Laserstrahl nach verschiedenen Zeitspannen beleuchtet. Die Aktivität der Proben wurde, wie vorstehend beschrieben, gemessen (Wallenfells, K. 1962, Methods Enzymol. 5:212-219).

Die Aktivität der Beta-Galactosidase nach CALI wurde als Funktion der zeit verfolgt. Die Aktivität wurde als Einheiten gemessen, die dem Test entsprechen und als Verhältnis zu der Aktivität der unbehandelten Beta-Galactosidase ausgedrückt sind.

| CALI von Beta-Galactosidase | | |
|---|---|---|
| Zeit (min) | % aktive Beta-Galactosidase | |
| | mit CALI | ohne CALI |
| 0 | 100 | 100 |
| 1 | 100 | 100 |
| 3 | 75 | 100 |
| 5 | 22 | 100 |
| 10 | 5 | 100 |
| 30 | <1 | 100 |

### Beispiel 3

### Gentechnische Erzeugung von scFv für die selektive Markierung mit Fluorescein und Malachitgrün und dessen Verwendung in der CALI

Um die Beschränkung einer nicht vorhersagbaren Markierung von ScFv mit dem Chromophor zu umgehen, wurde eine Folge von Lys-Resten gentechnisch an die folgenden Stellen in den scFv oder den Fabs eingefügt: (1) N-Terminus, (2) C-Terminus, (3) Linkerregion des scFv. Dabei werden Standard-Gensyntheseverfahren, die einem Fachmann auf dem Gebiet bekannt sind (Prodromou, C. & Pearl, L.H. 1992, Prot. Eng. 5: 827-829), verwendet. Nach der Markierung mit dem Chromophor wie in Beispiel 1 ii. beschrieben, wurde die Anwesenheit des Chromophors nachgewiesen und mittels UV-Absorption wie vorstehend beschreiben quantitativ bestimmt. Ferner wurden die rekombinanten scFvs, die mit dem Chromophor markiert waren, auf die Bindung an β-Gal mittels ELISA wie vorstehend beschrieben getestet. Diese scFvs wurden dann mittels CALI wie in Beispiel 1 iii. beschrieben getestet.
Die gentechnisch erzeugten, mit dem Chromophor markierten scFvs behielten ihre Bindungsfähigkeit an β-Gal und zeigten in den CALI-Experimenten eine mehr als 95%-ige Inaktivierung der β-Galactosidaseaktivität.

## Patentansprüche

1. Verfahren zur Identifikation der Funktion eines Liganden L, wobei die chromophor-unterstützte Laserinaktivierung (CALI) verwendet wird, gekennzeichnet durch die Stufen:
a) Auswählen eines Ligandenbindungspartners (LBP) mit Spezifität für den Liganden L,
b) Koppeln des LBP an einen laseraktivierbaren Marker (Tag) unter Bildung von LBP-Tag, gegebenenfalls nach vorheriger Modifikation des LBP im Hinblick auf eine effizientere Bindung an den Marker,
c) Inkontaktbringen des Liganden L mit mindestens einem LBP-Tag unter Bildung eines L/LBP-Tag-Komplexes, und
d) Bestrahlen des L/LBP-Tag-Komplexes mit einem Laserstrahl,
wobei der bestrahlte LBP-Tag selektiv den gebundenen Liganden modifiziert,
wobei die Reihenfolge der Stufen b) und c) vertauscht werden können.

2. Verfahren nach Anspruch 1, dadurch gekennzeichnet, daß der LBP ausgewählt ist aus dsFv, scFv, Fab, Diabody, immunglobulinartigen Molekülen, Peptiden, RNA, DNA, PNA, und kleinen organischen Molekülen, ausgenommen intakte Antikörpermoleküle.

3. Verfahren nach einen der Ansprüche 1 oder 2, dadurch gekennzeichnet, daß der LBP aus einer kombinatorischen Bank stammt, ausgenommen Hybridome.

4. Verfahren nach einem der Ansprüche 1 bis 3, dadurch gekennzeichnet, daß der laseraktivierbare Marker Malachitgrün, Fluorescein, Lissaminrhodamin, Tetramethylisothiocyanidrhodamin, Cyanin 3.18, AMCA-S, AMCA, BODIPY und Varianten davon, Cascade Blue, Cl-NERF, Dansyl, Dialkylaminocumarin, 4',5'Dichlor-2',7'-dimethoxyfluorescein, DM-NERF, Eosin, Eosin F3S, Erythrosin, Hydroxycumarin, Isosulfan blue, Lissamine Rhodamin B, Malachitgrün, Methoxycumarin, Naphthofluorescein, NBD, Oregon Green 488, 500, 514, PyMPO, Pyrol, Rhodamin 6G, Rhodamin Green, Rhodamin Red, Rhodol green, 2',4',5',7'-Tetrabromsulfonfluorescein, Tetramethylrhodamin, Texas Red oder X-Rhodamin ist.

5. Verfahren nach einem der Ansprüche 1 bis 4, dadurch gekennzeichnet, daß der LBP-Tag durch Anfügen von Lysinresten modifiziert wird.

6. Biologisches Molekül, erhalten nach einem Verfahren nach einem der Ansprüche 1 bis 5.

7. Vorrichtung zur Durchführung eines Verfahrens nach einem der Ansprüche 1 bis 5, dadurch gekennzeichnet, daß sie ein automatisiertes System aus integrierten unabhängigen Einheiten/Teilen zur Identifikation der Proteinfunktion ist und folgende Bestandteile umfaßt:
- eine automatisierte LBP-Screeningmaschine zur Erzeugung spezifischer LBPs, die gegen spezielle Zielmoleküle/Liganden gerichtet sind,
- eine Chromophor-Synthesevorrichtung zur Produktion von Chromophoren,
- eine LBP-Chromophor-Kopplungsvorrichtung zur Verknüpfung der der ausgewählten LBPs und der synthetisierten Chromophore,
- eine Beladungsvorrichtung zur Überführung des LBP-Tag in vorbestimmte Kavitäten, die mit dem Zielmolekül/Liganden in der Assay-Platform beschichtet sind,
- einen Transferroboterarm zur Bewegung der Assay-Platform in das Laser-System,
- eine Vorrichtung zum Ablesen der Aktivität,
- eine Datenbank,
- einen Zentralcomputersystem.

8. Verwendung eines LBP-Tags zur Durchführung eines Verfahrens zur Identifikation der Funktion eines Liganden L unter Verwendung der chromophor-unterstützten Laserinaktivierung.
